# EUROPEAN PATENT APPLICATION

(11) **EP 4 652 984 A1**
(43) Date of publication of application: **26.11.2025**
(21) Application number: 24177957.8
(22) Date of filing: 24.05.2024
(51) Int. Cl.: A61K 8/34, A61Q 5/10, A61K 8/46, A61K 8/31, A61K 8/44, A61K 8/73

(54) **DISCOLORING COMPOSITION FOR KERATIN FIBERS DYED WITH OXIDATIVE DYES OR DIRECT DYES**

(71) Applicant: Kao Corporation, Tokyo 103-8210 (JP)
(72) Inventor: Balke, Axel, 64297 Darmstadt (DE); Bauer, Peter, 64297 Darmstadt (DE)
(74) Representative: Hoffmann Eitle

(57) **Abstract**

A powder composition A for discoloring keratin fibers dyed with oxidative dyes and/or direct dyes, preferably human keratin fibers, more preferably human hair, comprising a) one or more organic sulfinic acid(s), and/or their salt(s), and/or their mixtures, b) one or more lipophilic compound(s) being liquid at 25°C and atmospheric pressure, wherein the total concentration of one or more compound(s) according to group b) is in the range of 0.1 % to 20% by weight, calculated to the total weight of the composition A.

## Description

### Field of the invention

The present invention is directed to a cosmetic composition for discoloring keratin fibers having been dyed with oxidative dyes or directed dyes. A kit-of-parts and bleaching/decoloring method is disclosed.

### Background of the invention

Consumers demand long-lasting high-performance products for perfectly covering gray hair. Such products typically comprise oxidative hair dyes. However, fashion trends are quickly changing, and the long lastingness of oxidative hair dyes may be perceived as a disadvantage for customers desiring a new look instantly. Therefore, there is a need for products being able to remove oxidative and direct hair dyes.

Conventional color removing products use the so-called "bleach wash" method, i.e., the oxidative destruction of oxidative hair dyes with the help of persulfates and peroxides. Unfortunately, these techniques confer a high degree of damage to the hair fibers. Moreover, these techniques always lead to significant lightening of the hair because the natural melanin is destroyed along with the artificial dyes.

Contrary to the oxidative color removal, reductive color removal techniques use reducing agents which are more selective to artificial dyes. The multimeric oxidative dyes within the hair fibers are reduced to monomers and can consequently be washed out of the fibers due to the smaller molecular weight of monomers. The oxidative damage is low and there is no destruction of the natural melanin. Thus, no additional lightening of the hair appears.

WO2012069599 discloses an anhydrous paste composition for removing color from dyed hair. However, pastes are not easily dispensed from containers. In addition, accurate weighing of pastes is difficult for the user.

Therefore, there is a real need to develop reductive color removing compositions with improved performance that are easy to dispense, weigh and handle.

### Summary of the invention

The first object of the present invention is a powder composition A for discoloring keratin fibers dyed with oxidative dyes and/or direct dyes, preferably human keratin fibers, more preferably human hair, comprising:
a) one or more organic sulfinic acid(s), and/or their salt(s), and/or their mixtures,
b) one or more lipophilic compound(s) being liquid at 25°C and atmospheric pressure,
wherein the total concentration of one or more compound(s) according to group b) is in the range of 0.1 % to 20% by weight, calculated to the total weight of the composition A.

The second object of the present invention is a kit-of-parts for discoloring keratin fibers dyed with oxidative dyes and/or direct dyes, preferably human keratin fibers, more preferably human hair, comprising:
- a composition A as defined above,
- an aqueous composition B having a pH in the range of 1 to 9,
wherein the compositions A and B are kept separate until immediately prior to application onto keratin fibers.

The third object of the present invention is a method for discoloring keratin fibers dyed with oxidative dyes and/or direct dyes, preferably human keratin fibers, more preferably human hair, comprising the following steps:
i) providing the composition A as defined above and dispersing and/or dissolving it in the composition B as defined above to yield an aqueous ready-to-use discoloring composition having a pH in the range of 1 to 9,
ii) applying the ready-to-use discoloring composition onto keratin fibers and leaving it for a time period in the range of 1 to 60 min,
iii) rinsing-off the ready-to-use discoloring composition with water and optionally drying the keratin fibers.

### Detailed description of the invention

The inventors of the present invention have surprisingly and unexpectedly found that the powder compositions, kits and methods according to the claims of the present invention yield a discoloring product with superior discoloration effect on keratin fibers dyed with oxidative and/or direct dyes, while also having a reduced sulfur-specific smell. The handling of the powder composition was excellent, and the dispensing was easy. The containers filled with the powder compositions were also easy to empty. In addition, washout of the dyes from the fibers was enhanced and damage to the fibers was minimal.

### Composition for discoloring dyed keratin fibers

The present invention is directed to a powder composition A for discoloring keratin fibers dyed with oxidative dyes and/or direct dyes, preferably human keratin fibers, more preferably human hair, comprising:
a) one or more organic sulfinic acid(s), and/or their salt(s), and/or their mixtures,
b) one or more lipophilic compound(s) being liquid at 25°C and atmospheric pressure,
wherein the total concentration of one or more compound(s) according to group b) is in the range of 0.1% to 20% by weight, calculated to the total weight of the composition A.

The composition of the present invention comprises one or more organic sulfinic acid(s), and/or their salt(s), and/or their mixtures, as compound(s) according to group a).

Suitably, one or more compound(s) according to group a) is according to the following general structure:

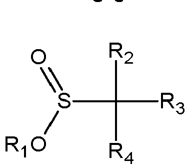

With the provision that R₁ is selected from H, NH₄⁺ or a monovalent metal cation, R₂ is selected from OH, NR₅R₆ in which R₅ and R₆, which may be identical or different, are chosen from H or C₁-C₆ alkyl, R₃ is selected from H, alkyl, alkenyl, cycloalkyl or aryl which is/are unsubstituted or substituted by 1 to 3 identical or different substituents chosen from OH, C₁-C₆ alkyl, O-C₁-C₆ alkyl, halogen or CF₃, R₄ is chosen from a radical COOR₁, SO₃R₁, COR₅, CONR₅R₆ or COOR₅ in which R₁, R₅, and R₆ have the denotations as above, or R₄ denotes H in case R₃ denotes an aryl group, in particular an aryl group substituted as described above, and/or their salt(s), and/or their mixtures. Suitable monovalent metal cations are all cosmetically acceptable cations such as sodium and potassium.

Suitable examples for compound(s) according to group a) are furyl-(amino)-methanesulfinic acid, amino-(thien-2-yl)-methanesulfinic acid, amino-(1H-imidazol-2-yl)-methanesulfinic acid, amino-(1,3-thiazol-2-yl)-methanesulfinic acid, amino-(1,3-oxazol-2-yl)-methanesulfinic acid, amino-(1H-pyrrol-2-yl)-methanesulfinic acid, amino-(hydroxyl-2-yl)-methanesulfinic acid, amino-(hydroxyl-4-yl)-methanesulfinic acid, amino-[3-hydroxy-5-(hydroxymethyl)-2-methylpyridin-4-yl]-methanesulfinic acid, amino-(quinolin-2-yl)-methanesulfinic acid, amino-(quinolin-4-yl)-methanesulfinic acid, 1H-Benzimidazol-2-yl-(amino)-methanesulfinic acid, 1,3-benzothiazol-2-yl-(amino)-methanesulfinic acid, 1,3-benzoxazol-2-yl-(amino)-methanesulfinic acid, hydroxy-(thien-2-yl)-methanesulfinic acid, hydroxy-(thien-3-yl)-methanesulfinic acid, 3-bromothien-2-yl)-(hydroxyl)-methanesulfinic acid, hydroxy-(1H-imidazol-2-yl)-methanesulfinic acid, hydroxy-(1H-imidazol-5-yl)-methanesulfinic acid, hydroxy-(1-methyl-5-nitro-1H-imidazol-2-yl)-methanesulfinic acid, hydroxy-(1,3-thiazol-2-yl)-methanesulfinic acid, hydroxy-(1,3-oxazol-2-yl)-methanesulfinic acid, hydroxy-(1H-pyrrol-2-yl)-methanesulfinic acid, hydroxy-(hydroxyl-2-yl)-methanesulfinic acid, hydroxy-(hydroxyl-4-yl)-methanesulfinic acid, hydroxy-[3-hydroxy-5-(hydroxymethyl)-2-methylpyridin-4-yl]-methanesulfinic acid, hydroxy-(quinolin-2-yl)-methanesulfinic acid, hydroxy-(quinolin-4-yl)-methanesulfinic acid, 1H-benzimidazol-2-yl-(hydroxyl)-methanesulfinic acid, 1,3-benzothiazol-2-yl-(hydroxyl)-methanesulfinic acid, 1,3-Benzoxazol-2-yl-(hydroxy)-methanesulfinic acid, 1,2-dihydroxyethane-1,2-disulfinic acid, 1,3-dihydroxypropane-1,3-disulfinic acid, hydroxy-{4-[hydroxy(sulfino)methyl]-phenyl}-methanesulfinic acid, {2-chloro-3-[hydroxy-(sulfino)-methyl]-cyclohexyl}-(hydroxy)-methanesulfinic acid, {2-chloro-3-[hydroxyl(sulfino)methyl]cyclopentyl}-(hydroxyl)-methanesulfinic acid, Hydroxy-{5-[hydroxy-(sulfino)-methyl]thien-2-yl}-methanesulfinic acid, hydroxy-{2-[hydroxy(sulfino)-methyl]phenyl}-methanesulfinic acid, hydroxy-{3-[hydroxy-(sulfino)-methyl]-phenyl}-methanesulfinic acid, 1,5-dihydroxypentane-1,5-disulfinic acid, 4-hydroxy-4-sulfinobutanoic acid, 1-Hydroxy-4-methoxy-4-oxobutane-1-sulfinic acid, 1-Hydroxy-4-ethoxy-4-oxobutane-1-sulfinic acid, 4-Hydroxy-4-sulfinopentanoic acid, 2-Hydroxy-5-methoxy-5-oxopentane-2-sulfinic acid, 2-Hydroxy-5-ethoxy-5-oxopentane-2-sulfinic acid, 4-Hydroxy-4-sulfinobut-2-enoic acid, 1-Hydroxy-4-methoxy-4-oxobut-2-ene-1-sulfinic acid, 1-Hydroxy-4-ethoxy-4-oxobut-2-ene-1-sulfinic acid, 4-hydroxy-4-sulfinopent-2-enoic acid, 2-Hydroxy-5-methoxy-5-oxopent-3-ene-2-sulfinic acid, 2-Hydroxy-5-ethoxy-5-oxopent-3-ene-2-sulfinic acid, 5-Hydroxy-5-sulfinopentanoic acid, 1-Hydroxy-5-methoxy-5-oxopentane-1-sulfinic acid, 1-Hydroxy-5-ethoxy-5-oxopentane-1-sulfinic acid, 5-hydroxy-5-sulfinohexanoic acid, 2-Hydroxy-6-methoxy-6-oxohexane-2-sulfinic acid, 2-Hydroxy-6-ethoxy-6-oxohexane-2-sulfinic acid, 4-[Hydroxy-(sulfino)-methyl]-benzoic acid, 4-[amino-(sulfino)-methyl]-benzoic acid, {4-[Hydroxy-(sulfino)-methyl]-phenoxy}-acetic acid, {4-[amino-(sulfino)-methyl]-phenoxy}-acetic acid, 3-Hydroxy-4-[hydroxy-(sulfino)-methyl]-benzoic acid, 3-Hydroxy-4-[amino-(sulfino)-methyl]-benzoic acid, (4-cyanophenyl)-(hydroxy)-methanesulfinic acid, [(2-Methoxy-2-oxoethyl)-amino]-(oxo)-methane-sulfinic acid, 2-Hydroxy-2-sulfinoacetic acid, 2-amino-2-sulfino-acetic acid, (4-cyanophenyl)-(amino)-methane-sulfinic acid,(4-nitrophenyl)-(hydroxy)-methane-sulfinic acid, (4-nitrophenyl)-(amino)-methane-sulfinic acid, 4-hydroxy-1,1-dimethyl-4-sulfinopiperidinium, 1-allyl-4-hydroxy-1-methyl-4-sulfinopiperidinium, 2-Hydroxy-2-sulfino-propionic acid, 2-amino-2-sulfino-propionic acid, 2-Hydroxy-2-sulfinato-acetic acid ethyl ester, 2-Amino-2-sulfinato-acetic acid ethyl ester, 4-Hydroxy-1,1-dimethylpiperidinium-4-sulfinate,1-Allyl 4-hydroxy-1-methylpiperidinium-4-sulfinate, 1-Hydroxy-2-(trimethylammonio)-ethane-sulfinate, Hydroxy-[4-(trimethylammonio)-phenyl]-methane-sulfinate, 4-[Hydroxy-(sulfino)-methyl]-benzene-sulfonic acid, 2-[Hydroxy-(sulfino)-methyl]-benzene-sulfonic acid, 3-Hydroxy-3-sulfinato-propionic acid, 3-amino-3-sulfinato-propionic acid, 1-Hydroxy-2-methoxy-2-oxoethanesulfinic acid, 2-amino-1-hydroxy-2-oxoethanesulfinic acid, 2-(dimethylamino)-1 -hydroxy-2-oxoethanesulfinic acid, hydroxy-(sulfino)-methanesulfonic acid, Hydroxy-(phenyl)-methane-sulfinic acid, hydroxy-(4-hydroxyphenyl)-methanesulfinic acid, hydroxy-(4-methoxyphenyl)-methane-sulfinic acid, and/or their salt(s), and/or their mixtures.

Preferably, form the viewpoint of reducing power, one or more compound(s) according to group a) has the following structure:

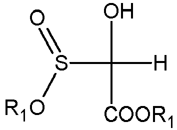

With R₁ having the same denotation as above.

The most preferred compound(s) according to group a) is/are 2-hydroxy-2-sulfinoacetic acid, and/or its salts, preferably it is disodium-2-hydroxy-2-sulfinatoacetate.

It is preferred from the viewpoint of color removal that the total concentration of one or more compound(s) according to group a) is in the range of 0.1% to 90% by weight, preferably in the range of 0.25% to 80% by weight, more preferably in the range of 0.5% to 70% by weight, still more preferably in the range of 1% to 60% by weight, still more preferably in the range of 3% to 55% by weight, calculated to the total weight of the composition A.

The composition A comprises one or more lipophilic compound(s) being liquid at 25°C and atmospheric pressure as compound(s) according to group b).

Preferably, the one or more compound(s) according to group b) are one or more vegetable or mineral oil(s), C₁₂ to C₂₂ fatty alcohols, esters of C₃ to C₂₂ alcohols with C₁₂ to C₂₂ fatty acids, silicone(s), and/or their mixtures.

Suitable silicones are dimethylpolysiloxanes, and modified silicones (for example, amino-modified silicones, fluorine-modified silicones, alcohol-modified silicones, polyether-modified silicones, epoxy-modified silicones, or alkyl-modified silicones), but dimethylpolysiloxane, polyether-modified silicones and amino-modified silicones are preferred. Amino-modified silicones are commonly known under their CTFA name amodimethicone.

Specific examples of suitable commercially available amodimethicone oils such as SF8452C, SS-3551 (all by Dow Corning Toray Co., Ltd.), KF-8004, KF-867S, and KF-8015 (all by Shin-Etsu Chemical Co. , Ltd.), and amodimethicone emulsions such as SM8704C, SM8904, BY22-079, FZ-4671, and FZ-4672 (all by Dow corning Toray Co., Ltd.).

The amino-modified silicone may be any silicone having an amino group or a quaternary ammonium group, and examples thereof include amine-modified silicone oil having all or a part of the terminal hydroxyl groups capped with a methyl group for example, and an amodimethicone which does not have the terminals capped.

From the viewpoint of cosmetic acceptance, it is further preferred that the one or more compound(s) according to group b) are castor oil, olive oil, safflower oil, shea butter oil, argan oil, almond oil, avocado oil, ricinus oil, coconut oil, palm oil, sesame oil, peanut oil, sunflower oil, peach kernel oil, wheat germ oil, macadamia nut oil, night primrose oil, jojoba oil, soya oil, passiflora oil, black cumin oil, borage oil, grapeseed oil, macadamia oil, rosehip oil, hempseed oil, mineral oil, paraffinum perliquidium, paraffinum subliquidum, polydecenes, C₁₃-C₁₅ alkanes, C₁₅-C₁₇ alkanes, C₁₈-C₂₀ alkanes, lauryl alcohol, octyldodecanol, isopropylmyristate, isopropylpalmitate, silicones such as aminated or non-aminated silicones, and/or their mixtures.

More preferably, said compounds are octyldodecanol, C₁₃-C₁₅ alkanes, C₁₅-C₁₇ alkanes, C₁₈-C₂₀ alkanes castor oil, mineral oil, isopropyl myristate, and/or their mixtures.

The most preferred compound according to group b) is octyldodecanol.

The composition A comprises the one or more compound(s) according to group b) at a total concentration in the range of 0.1% to 20% by weight, calculated to the total weight of the composition A.

It is preferred from the viewpoint of smell-reduction that the total concentration of compound(s) according to group b) is in the range of 0.25% to 15% by weight, preferably in the range of 0.5% to 12% by weight, more preferably in the range of 0.75% to 12% by weight, still more preferably in the rage of 1% to 12 % by weight, still more preferably in the range of 3% to 12% by weight, calculated to the total weight of the composition A.

The composition A is a powder composition.

A powder composition within the meaning of the present invention is any solid composition at 25°C and atmospheric pressure that consists of freely flowing individual particles/aggregates. The presence of the compounds according to group b) does not alter the overall state of a powder.

Composition A may also optionally comprise water at 10% by weight or less, preferably at 1% by weight or less, still more preferably at 0.1% by weight or less, calculated to the total weight of the composition A, still more preferably the composition A is anhydrous. However, the presence of water does not change the overall state of a powder.

In addition, the composition A may optionally comprise one or more pulverulent excipient(s) as compound(s) according to group d), preferably one or more compound(s) according to group d) is cellulose, chemically modified cellulose fibers, lignin fibers, pectin fibers, natural starch(es), chemically modified starch(es), hydrolyzed natural starch(es), diatomaceous earth, kaolin, bentonite, nylon powder, montmorillonit, gypsum, sawdust and perlite, and/or their mixtures, more preferably one or more compound(s) according to group d) is a natural starch.

Suitable natural starches are potato starch, rice starch, corn starch, tapioca starch, wheat starch, and barley starch, and/or their mixtures.

In case the composition A comprises one or more compound(s) according to group d), it is preferred that the total concentration of one or more compound(s) according to group d) is 10% by weight, preferably 20% by weight, more preferably 30% by weight, calculated to the total weight of the composition A.

It is further preferred from the viewpoint of stability and acidity that the composition of the present invention comprises one or more sulfonic acid compound according to the following general structure:

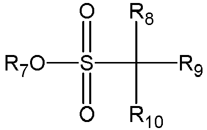

With the provision that R₇ is selected from H, NH₄⁺ or a monovalent metal cation, R₈ is selected from OH, NR₁₁R₁₂ in which R₁₁ and R₁₂, which may be identical or different, are chosen from H or C₁-C₆ alkyl, R₉ is selected from H, alkyl, alkenyl, cycloalkyl or aryl which is/are unsubstituted or substituted by 1 to 3 identical or different substituents chosen from OH, C₁-C₆ alkyl, O-C₁-C₆ alkyl, halogen or CF₃, R₁₀ is chosen from a radical COOR₇, SO₃R₇, COR₁₁, CONR₁₁R₁₂ or COOR₁₁ in which R₇, R₁₁ and R₁₂ have the denotations as above, or R₁₀ denotes H when R₉ denotes an aryl group, in particular an aryl group substituted as described above, and/or their salt(s), and/or their mixtures, preferably one or more said compound(s) have the following structure:

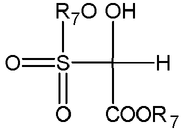

With R₇ having the same denotation as above, more preferably one or more sulfonic acid compound is disodium-2-hydroxy-2-sulfonatoacetate.

When the sulfonic acid compounds are present, it is preferred that the total concentration of sulfonic acid compounds is in the range of 0.1% to 50% by weight, calculated to the total weight of the composition.

It is further preferred that the composition of the present invention comprises one or more organic reducing agent(s) having at least one thiol group(s), and/or their salt(s), and/or their mixtures, as compound(s) according to group c), and preferably the molar ratio of compound(s) according to group a) to compound(s) according to group c) is in the range of 10:0.1 to 0.1:10, preferably in the range of 3:1 to 1:4, more preferably it is in the range of 1.5:1 to 1:1.5.

The term 'thiol group' has its common meaning in the field of chemistry, i.e., it is an unmodified thiol group able to act as reducing moiety within the molecule of the organic compound.

Suitably, the one or more compound(s) according to group c) is/are cysteine, cystine, cysteamine, thioglycolic acid, thiolactic acid, and/or their salt(s), and/or their mixtures, preferably it is cysteine and/or its salt(s), from the viewpoint of commercial availability.

### Surfactants

Preferably, the composition according to the present invention comprises one or more surfactant(s) as compound(s) according to group d), preferably one or more non-ionic, anionic, cationic or amphoteric/zwitterionic surfactant surfactant(s), and/or their salt(s), and/or their mixtures.

Anionic surfactants suitable are in principle known from the cleansing compositions. These are anionic surfactants of the sulfate, sulfonate, carboxylate and alkyl phosphate type, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates. Preferred anionic surfactants are alkyl sulphate surfactants especially lauryl sulphate and its salts.

Suitable amphoteric/zwitterionic surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and - acetate are also suitable.

Typical cationic surfactants are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The preferred surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics^{R}", as well as ethoxylated fatty alcohols, C₁₀-C₂₂-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Preferably, the one or more non-ionic surfactant(s) are selected from alkyl polyglycosides, ethoxylated triglycerides, ethoxylated fatty alcohols, ethoxylated fatty acid esters, and/or their mixtures, still more preferably said compound(s) are selected from ethoxylated C₁₀-C₂₂ fatty alcohols.

It is further preferred that the concentration one or more compound(s) according to group d), preferably the total concentration of non-ionic surfactant(s), still more preferably the total concentration of ethoxylated C₁₀-C₂₂ fatty alcohols, is in the range from 0.1% to 25% by weight, preferably in the range of 0.25% to 20% by weight, more preferably in the range of 0.5% to 15% by weight, still more preferably in the range of 1% to 10% by weight, calculated to the total weight of the composition.

In a further preferred aspect, the composition of the present invention is an emulsion, preferably an oil-in-water emulsion. Suitably, the compounds according to group d) as disclosed above are present.

### Thickeners

The compositions A and/or B may comprise one or more thickening agent(s), preferably one or more thickening polymer(s), more preferably one or more non-ionic, anionic, cationic, or amphoteric/zwitterionic thickening polymer(s).

Suitable anionic thickening polymers are copolymers and/or crosspolymers which comprise an acrylate and/or methacrylate monomer unit and optionally least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer, Hydroxyethyl acrylate / sodium acryloyldimethyl taurate copolymer, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

Suitable non-ionic thickening polymers are, for example, alkyl modified cellulose derivatives such as (C₂-C₈)-alkylcellulose, cellulose polymers, preferably methyl cellulose, ethyl cellulose, hydroxyethylcellulose, methylhydroxyethylcellulose, methylhydroxypropylcellulose, or starch based polymers such as vegetable starch and/or their synthetically modified derivatives such as hydroxypropyl starch phosphate.

Suitable cationic non-associative polymers are Polyquaternium 6, Polyquaternium 16, and Polyquaternium 37.

The most preferred thickening polymers of the composition of the present invention are Carbomer and xanthan gum.

The preferred concentration of thickening agents, preferably of the thickening polymers, is in the range of 0.1% to 10% by weight, calculated to the total weight of each of the compositions A and/or B.

### Additional reducing agents

It is further preferred from the viewpoint of discoloring performance that the composition of the present invention comprises one or more additional reducing agent(s) different from the group(s) according to a) and c), preferably the one or more additional reducing agent(s) is ascorbic acid, and more preferably the total concentration of ascorbic acid is in the range of 0.1% to 10% by weight, calculated to the total weight of the composition.

### Kit-of-parts

The present invention is also directed to A kit-of-parts for discoloring keratin fibers dyed with oxidative dyes and/or direct dyes, preferably human keratin fibers, more preferably human hair, comprising:
- a composition A comprising one or more compound(s) according to group a) as defined above,
- an aqueous composition B comprising one or more compound(s) according to groups b) as defined above at a total concentration of 10% by weight or more, calculated to the total weight of composition B,
wherein the compositions A and B are kept separate until immediately prior to application onto keratin fibers.

The term 'aqueous' has the same denotation as disclosed further above.

It is preferred from the viewpoint of cosmetic safety that the mixture of compositions A and B has a pH of 1 or more, preferably 2 or more, still more preferably 2.5 or more.

It is preferred from the viewpoint of stability that the mixture of compositions A and B has a pH of 6 or less, preferably 5 or less, still more preferably 4.5 or less.

For attaining the above-mentioned effects, it is preferred that the mixture of compositions A and B has a pH in the range of 1 to 6, more preferably in the range of 2 to 5, still more preferably in the range of 2.5 to 4.5.

The composition B may be water such as tap water.

From the viewpoint of cosmetic acceptance, it is preferred that the composition B further comprises one or more compound(s) according to group b) as defined above The total concentration of the one or more compound(s) according to group b) in the composition B is in the range of 0.1% to 10% by weight, calculated to the total weight of the composition B.

As the composition B is an aqueous composition, it is preferred that the composition B comprises water at 20% by weight or more, preferably at 30% by weight or more, still more preferably at 50% by weight or more, calculated to the total weight of the composition B.

Optionally, the composition B also comprises surfactants or thickening agents at the concentration range as disclosed above for the composition of the present invention.

### Method for discoloring

The present invention is also directed to a method for discoloring keratin fibers dyed with oxidative dyes and/or direct dyes, preferably human keratin fibers, more preferably human hair, comprising the following steps:
i) providing the composition A as defined above and dispersing and/or dissolving it in the composition B as defined in any of the claims 13 to 14 to yield an aqueous ready-to-use discoloring composition having a pH in the range of 1 to 9,
ii) applying the ready-to-use discoloring composition onto keratin fibers and leaving it for a time period in the range of 1 to 60 min,
iii) rinsing-off the ready-to-use discoloring composition with water and optionally drying the keratin fibers.

It is preferred from the viewpoint of reducing power that the pH of the ready-to-use discoloring composition of step i) is in the range of 1 to 7, more preferably in the range of 1.5 to 5.

The preferred application time if step ii) is in the range of 2 min to 45 min, more preferably it is in the range of 5 min to 35 min, still more preferably it is in the range of 10 min to 30 min.

The following examples are to illustrate the present invention and not to limit it.

### EXAMPLES

**Table 1 The following compositions A were prepared.**

| **Ingredients** | | **Inv. 1** | **Inv**. **2** | **Inv. 3** | **Comp.1** |
|---|---|---|---|---|---|
| | | **% by weight** | | | |
| a) | Disodium-2-hydroxy-2-sulfinatoacetate | 24.13 | 24.13 | 24.13 | 24.13 |
| c) | Disodium-2-hydroxy-2-sulfonatoacetate | 24.13 | 24.13 | 24.13 | 24.13 |
| b) | Octyldodecanol | 10.0 | 5.0 | - | - |
| b) | C₁₃-C₁₅ alkanes | - | - | 10.0 | - |
| d) | Cysteine HCl • 1 H₂0 | 38.25 | 38.25 | 38.25 | 38.25 |
| - | Fragrance, thickener | 0.4 | 0.4 | 0.4 | 0.4 |
| - | Xanthan gum | 2.5 | 2.5 | 2.5 | 2.5 |

As composition B, tap water was used.

Compositions A and B were mixed in a weight ratio of 1:10. 5 hairdressers were asked to rate the odour of the ready-to-use composition during preparation and application to the hair for 25 minutes. They were asked to assign an integer number on a scale from 5 (no smell) to 1 (strong smell), without being informed about the type of product used. The arithmetic mean of their scores is reported below.

The results of the olfactory evaluation were as follows:

**Table 2**

| | | | | |
|---|---|---|---|---|
| **Smell rating** | **Inv. 1** | **Inv. 2** | **Inv. 3** | **Comp. 1** |
| | 4.5 | 4.0 | 4.5 | 2.5 |

Comparative example 1 did not mix with composition B, i.e., the preparation of a ready-to-use mixture was not possible.

## Claims

1. A powder composition A for discoloring keratin fibers dyed with oxidative dyes and/or direct dyes, preferably human keratin fibers, more preferably human hair, comprising:
a) one or more organic sulfinic acid(s), and/or their salt(s), and/or their mixtures,
b) one or more lipophilic compound(s) being liquid at 25°C and atmospheric pressure,
wherein the total concentration of one or more compound(s) according to group b) is in the range of 0.1% to 20% by weight, calculated to the total weight of the composition A.

2. The composition according to claim 1 **characterized in that** one or more compound(s) according to group a) have the following general structure: 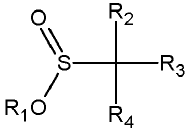 With the provision that R₁ is selected from H, NH₄⁺ or a monovalent metal cation, R₂ is selected from OH, NR₅R₆ in which R₅ and R₆, which may be identical or different, are chosen from H or C₁-C₆ alkyl, R₃ is selected from H, alkyl, alkenyl, cycloalkyl or aryl which is/are unsubstituted or substituted by 1 to 3 identical or different substituents chosen from OH, C₁-C₆ alkyl, O-C₁-C₆ alkyl, halogen or CF₃, R₄ is chosen from a radical COOR₁, SO₃R₁, COR₅, CONR₅R₆ or COOR₅ in which R₁, R₅, and R₆ have the denotations as above, or R₄ denotes H in case R₃ denotes an aryl group, in particular an aryl group substituted as described above, and/or their salt(s), and/or their mixtures, preferably one or more compound(s) according to group a) has the following structure: 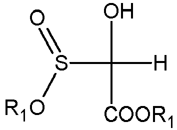 With R₁ having the same denotation as above, more preferably one or more compound(s) according to group a) is 2-hydroxy-2-sulfinoacetic acid, and/or its salts, still more preferably it is disodium-2-hydroxy-2-sulfinatoacetate.

3. The composition according to any of the preceding claims **characterized in that** the total concentration of one or more compound(s) according to group a) is in the range of 0.1% to 90% by weight, preferably in the range of 0.25% to 80% by weight, more preferably in the range of 0.5% to 70% by weight, still more preferably in the range of 1% to 60% by weight, still more preferably in the range of 3% to 55% by weight, calculated to the total weight of the composition A.

4. The composition according to any of the preceding claims **characterized in that** the one or more compound(s) according to group b) are one or more vegetable or mineral oil(s), C₁₂ to C₂₂ fatty alcohols, esters of C₃ to C₂₂ alcohols with C₁₂ to C₂₂ fatty acids, silicone(s), and/or their mixtures, preferably said compounds are castor oil, olive oil, safflower oil, shea butter oil, argan oil, almond oil, avocado oil, ricinus oil, coconut oil, palm oil, sesame oil, peanut oil, sunflower oil, peach kernel oil, wheat germ oil, macadamia nut oil, night primrose oil, jojoba oil, soya oil, passiflora oil, black cumin oil, borage oil, grapeseed oil, macadamia oil, rosehip oil, hempseed oil, mineral oil, paraffinum perliquidium, paraffinum subliquidum, polydecenes, C₁₃-C₁₅ alkanes, C₁₅-C₁₇ alkanes, C₁₈-C₂₀ alkanes, lauryl alcohol, octyldodecanol, isopropylmyristate, isopropylpalmitate, silicones such as aminated or non-aminated silicones, and/or their mixtures, more preferably said compounds are octyldodecanol, C₁₃-C₁₅ alkanes, C₁₅-C₁₇ alkanes, C₁₈-C₂₀ alkanes castor oil, mineral oil, isopropyl myristate, and/or their mixtures, still more preferably one or more compound(s) according to group b) is octyldodecanol.

5. The composition according to any of the preceding claims **characterized in that** the total concentration of compound(s) according to group b) is in the range of 0.25% to 15% by weight, preferably in the range of 0.5% to 12% by weight, more preferably in the range of 0.75% to 12% by weight, still more preferably in the rage of 1% to 12 % by weight, still more preferably in the range of 3% to 12% by weight, calculated to the total weight of the composition A.

6. The composition according to any of the preceding claims **characterized in that** the composition A further comprises one or more sulfonic acid compound as compound(s) according to group c) according to the following general structure: 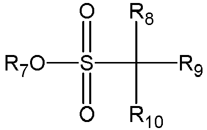 With the provision that R₇ is selected from H, NH₄⁺ or a monovalent metal cation, R₈ is selected from OH, NR₁₁R₁₂ in which R₁₁ and R₁₂, which may be identical or different, are chosen from H or C₁-C₆ alkyl, R₉ is selected from H, alkyl, alkenyl, cycloalkyl or aryl which is/are unsubstituted or substituted by 1 to 3 identical or different substituents chosen from OH, C₁-C₆ alkyl, O-C₁-C₆ alkyl, halogen or CF₃, R₁₀ is chosen from a radical COOR₇, SO₃R₇, COR₁₁, CONR₁₁R₁₂ or COOR₁₁ in which R₇, R₁₁ and R₁₂ have the denotations as above, or R₁₀ denotes H when R₉ denotes an aryl group, in particular an aryl group substituted as described above, and/or their salt(s), and/or their mixtures, preferably one or more said compound(s) have the following structure: 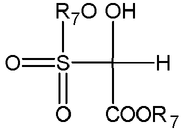 With R₇ having the same denotation as above, more preferably one or more sulfonic acid compound is disodium-2-hydroxy-2-sulfonatoacetate.

7. The composition according to any of the preceding claims **characterized in that** the composition A comprises water at 10% by weight or less, preferably at 1% by weight or less, still more preferably at 0.1% by weight or less, calculated to the total weight of the composition A, still more preferably the composition A is anhydrous.

8. The composition according to any of the preceding claims **characterized in that** the composition A further comprises one or more organic reducing agent(s) having at least one thiol group(s), and/or their salt(s), and/or their mixtures, as compound(s) according to group c), and preferably the molar ratio of compound(s) according to group a) to compound(s) according to group d) is in the range of 10:0.1 to 0.1:10, preferably in the range of 3:1 to 1:4, more preferably it is in the range of 1.5:1 to 1:1.5, preferably the one or more compound(s) according to group c) is/are cysteine, cystine, cysteamine, thioglycolic acid, thiolactic acid, and/or their salt(s), and/or their mixtures, preferably it is cysteine and/or its salt(s).

9. The composition according to any of the preceding claims **characterized in that** the composition A further comprises one or more surfactant(s) as compound(s) according to group e), preferably one or more non-ionic, anionic, cationic or amphoteric/zwitterionic surfactant surfactant(s), and/or their salt(s), and/or their mixtures, more preferably one or more non-ionic surfactant(s), said one or more non-ionic surfactant(s) preferably being alkyl polyglycosides, ethoxylated triglycerides, ethoxylated C₁₀-C₂₂ fatty alcohols, ethoxylated fatty acid esters, and/or their mixtures, still more preferably said compound(s) are selected from ethoxylated fatty alcohols.

10. The composition according to claim 9 **characterized in that** the total concentration of one or more compound(s) according to group e), preferably the total concentration of non-ionic surfactant(s), still more preferably the total concentration of ethoxylated fatty alcohols, is in the range from 0.1% to 25% by weight, preferably in the range of 0.25% to 20% by weight, more preferably in the range of 0.5% to 15% by weight, still more preferably in the range of 1% to 10% by weight, calculated to the total weight of the composition.

11. The composition according to any of the preceding claims **characterized in that** the composition A further comprises one or more pulverulent excipient(s) as compound(s) according to group f), preferably one or more compound(s) according to group d) is cellulose, chemically modified cellulose fibers, lignin fibers, pectin fibers, natural starch(es) such as potato starch, rice starch, corn starch, tapioca starch, wheat starch, and barley starch, chemically modified starch(es), hydrolyzed natural starch(es), diatomaceous earth, kaolin, bentonite, nylon powder, montmorillonit, gypsum, sawdust and perlite, and/or their mixtures, more preferably one or more compound(s) according to group d) is a natural starch.

12. The composition according to claim 11 **characterized in that** the total concentration of one or more compound(s) according to group f) is 1% by weight or more, preferably 10% by weight or more, more preferably 20% by weight more, still more preferably 30% by weight more, calculated to the total weight of the composition A.

13. A kit-of-parts for discoloring keratin fibers dyed with oxidative dyes and/or direct dyes, preferably human keratin fibers, more preferably human hair, comprising:
- a composition A as defined in any of the claims 1 to 12,
- an aqueous composition B having a pH in the range of 1 to 9,
wherein the compositions A and B are kept separate until immediately prior to application onto keratin fibers.

14. The kit-of-parts according to claim 13 **characterized in that** the mixture of the compositions A and B has a pH in the range of 1 to 6, more preferably in the range of 2 to 5, still more preferably in the range of 2.5 to 4.5.

15. A method for discoloring keratin fibers dyed with oxidative dyes and/or direct dyes, preferably human keratin fibers, more preferably human hair, comprising the following steps:
i) providing the composition A as defined in any of the claims 1 to 12 and dispersing and/or dissolving it in the composition B as defined in any of the claims 13 to 14 to yield an aqueous ready-to-use discoloring composition having a pH in the range of 1 to 9,
ii) applying the ready-to-use discoloring composition onto keratin fibers and leaving it for a time period in the range of 1 to 60 min,
iii) rinsing-off the ready-to-use discoloring composition with water and optionally drying the keratin fibers.
